# EUROPEAN PATENT SPECIFICATION

(11) **EP 1 265 070 B1**
(45) Date of publication and mention of the grant of the patent: **08.03.2006**
(21) Application number: 01112916.0
(22) Date of filing: 05.06.2001
(51) Int. Cl.: G01N 33/543

(54) **Chip exposing plant extracts on its surface**
Chip mit Pflanzenextrakten auf der Oberfläche
Puce exposant sur sa surface des extraits de plantes

(43) Date of publication of application: 11.12.2002
(73) Proprietor: Advanced Gene Technology, Corp., 407 Taichung (TW)
(72) Inventor: Chang, Su-Chen, 406 Taichung (TW); Hsu, Li-Wei, 406 Taichung (TW); Chen, Jyh-Phen, Taipei (TW); Lee, Jeng-Woei, Yung He City, Taipei (TW)
(74) Representative: Albrecht, Thomas

(56) References cited:
- WO-A-00/16062
- WO-A-01/14425
- WO-A-01/33193
- WO-A-98/20163
- WO-A-99/38013
- PAWELETZ CLOUD P ET AL: "Reverse phase protein microarrays which capture disease progression show activation of pro-survival pathways at the cancer invasion front." ONCOGENE, vol. 20, no. 16, 2001, pages 1981-1989, XP001021583 ISSN: 0950-9232
- LUEKING A ET AL: "PROTEIN MICROARRAYS FOR GENE EXPRESSION AND ANTIBODY SCREENING" ANALYTICAL BIOCHEMISTRY, ACADEMIC PRESS, SAN DIEGO, CA, US, vol. 270, no. 1, May 1999 (1999-05), pages 103-111, XP000949937 ISSN: 0003-2697

## Description

### Technical Field of the Invention

The present invention relates to the allocation of fractions or components obtained from herbs in microarrays on a plastic substrate. The plastic substrate containing allocated fractions or components of herbs is useful as a platform for screening for active ingredients contained in the herbs that have specific pharmacological or therapeutical functions.

### Background of the Invention

The use of herbs or an extract or fraction thereof as a medicine has been well known for so many years. For example, the bark of willow tree has been used as an antipyretic and analgesic agent for more than 2000 years, and an extract from the bark of Peruvian *Cinchona* has been used for the treatment of malaria since the 17^{th} century.

Up to date, however, only a few active ingredients contained in herbs that have specific pharmacological or therapeutical functions were discovered. For example, salicin, a salicyl alcohol glucoside, was recognized as an active ingredient in the bark of willow tree that exhibited antipyretic and analgesic properties in the middle of 20^{th} century, and quinine was recognized as an active ingredient in the bark of Peruvian *Cinchona* that exhibited an effect on the treatment of malaria in 1820.

The discovery of an active ingredient from a herb was laborious and time-consuming. For example, taxol, an anti-tumor drug, was isolated from the bark of North American yew tree *Taxus brevifolia,* after the work of screening of more than 110,000 samples derived from more than 35,000 plant genera collected worldwide. In general, the common strategy in the discovery of an active ingredient from a herb was to identify the function(s) of the herb in human body and to apply various physical and chemical procedures for isolating active fraction(s) of the herb and then for separating and purifying the active ingredient. There was no general guideline for the discovery of an active ingredient from a herb. Though the understanding of pathogeny in the molecular or gene level based on the development of pharmacology, cell biology and molecular biology was significantly increasing, and the shotgun screening of an extract of a herb for hitting desired active ingredient(s) contained in the herb was developed, however the discovery of an active ingredient from a herb was still a laborious, trial-and-error work and progressed in a slow pace.

There has been a demand in the art to develop a new tool to conduct a rapid screening for active ingredients from herbs or an extract or fraction thereof that have specific pharmacological or therapeutical functions.

The technique of immobilization of large biological molecules on a solid substrate (e.g. nylon membrane), such as Western blot for immobilizing peptides or proteins, Southern blot for immobilizing DNA fragments and Northern blot for immobilizing RNA fragments, was used in the art, and the immobilized molecules interacted with a labeled probe and then the resultant probe-labeled molecules were imaged. For example, enzyme-linked immunosorbent assay (ELISA) involved immobilizing large biological molecules on a substrate, interacting the immobilized molecules with a labeled substance, and then coloring and imaging the resultant labeled molecule(s). The ELISA could be performed on a conventional 96-well microplate.

Recently, the high-density gridding technology was used in the art for detecting the presence of a target material in biological samples (e.g. DNA or proteins), wherein the samples were immobilized in a predetermined array on a solid substrate (e.g. glass slide) and then were hybridized with a labeled probe, followed by washing and imaging. In applying the high-density gridding technology, the biological samples were known DNA or protein pools that were homogeneous, and the labeled probe was an unknown or unidentified substance that was heterogeneous. The advantage of the high-density gridding technology resided in that a tiny volume of a sample could be immobilized in a small gridded area on the surface of the substrate and thousands of samples could be manipulated simultaneously. Further, a computer-controlled three-axis robot and a unique pen tip assembly (i.e. microarrayer) could be used to generate the high-density, gridded arrays of biological samples on the surface of a substrate.

WO 01/33193 discloses an automated and validated method for preparing plant extracts for use in the high throughput screening of biological assays using a robotic sample processor for the aspiration and dispensing of solvent extracts and a fully automated solid phase extraction apparatus to produce column fractionated plant extracts. The method comprises the steps: (a) thoroughly extracting a biological source material with at least one solvent to produce at least one solvent extract containing the potentially bio-active biochemicals of the source material; (b) removing from the solvent extracts interferences, comprising non-selectively bio-active compounds that interfere with biological assays; (c) subjecting the solvent extracts to chromatography by an automated system for handling samples; (d) detecting the potentially selectively bio-active biochemicals of the source material in the eluent, and (e) collecting the biochemicals in isolated fractions having standardized concentrations under control by the automated system with associated data.

WO 01/14425 describes protein chips on which high density of protein probe arrays are fixed, a method for manufacturing the proteins chips, automized diagnostic systems comprising the protein chips and the use thereof. The highly integrated structure of the protein chip makes a biochemical or an immunological assay faster, suitable for automatization precise and easy to handle. The usage of the protein chip encompasses clinical diagnosis, researches for the kinetics of enzymatic reactions and screening antagonists or ligands which bind to the interested receptors. In particular, the protein chip enables multipurpose diagnosis of various diseases for a number of patients by a test.

So far, however, the high-density gridding technology was used for analyzing just macromolecules, such as proteins and nucleic acids, where the immobilized samples on the surface of a solid substrate (e.g. glass slide) were homogeneous. There was no teaching or suggestion in the art that heterogeneous samples containing large, biologically active molecules (such as proteins or nucleic acids) or small, biologically active molecules (such as secondary metabolites) could be immobilized on the surface of a solid substrate (e.g. plastic slide). There was also no teaching or suggestion in the art that homogeneous or heterogeneous samples obtained from herbs or extracts or fractions thereof could be immobilized on the surface of a solid substrate (e.g. plastic slide), especially in a microarray format. Furthermore, there was no teaching or suggestion in the art that by applying the high-density gridding technology, a high throughput screening for biologically active molecules from herbs could be conducted with the homogeneous or heterogeneous, unknown samples allocated on the surface of a solid substrate (e.g. plastic slide) that interacted with homogeneous or heterogeneous, known labeled probe(s).

Therefore, the present invention discloses a new platform for screening for active ingredients from herbs with homogeneous or heterogeneous, known labeled probe(s), which comprises homogeneous or heterogeneous ingredients from herbs that are immobilized on a solid substrate (plastic slide).

### Summary of the Invention

The present invention discloses a new platform, named herbal chip, comprising an herbal chip comprising a plastic slide, a coating on the plastic slide which binds fractions or components obtained from herbs to said slide in independently allocated microarrays on the coating wherein the plastic slide is pre-treated with a polyfunctional aldehyde followed by soaking in a solution of NH₂ group(s) - providing precursor before coating the plastic slide, and wherein said coating is made of a polyfunctional epoxide compound linking the components contained in herbs to the pre-treated plastic slide. The herbal chip of the present invention is useful for screening for active ingredients contained in the herbs that have specific pharmacological or therapeutical functions.

The present invention also discloses a method for producing the herbal chip, as defined in the claims.

Further, the present invention discloses a method of using the herbal chip for screening for active ingredients contained in herbs, as defined in the claims.

### Brief Description of the Figures

Figure 1 shows the perspective of a plastic slide for producing the herbal chip of the present invention.
Figure 2A shows an example of the herbal chip of the present invention where samples were gridded with 6 x 10 matrix on the plastic slide of Figure 1.
Figure 2B shows endogenous fluorescence of the gridded samples on the herbal chip shown in Figure 2A prior to hybridization with labeled probes.
Figure 2C shows fluorescence image of the gridded samples on the herbal chip shown in Figure 2A after hybridization with Cy3-labeled TNF-αR (tumor necrosis factor-alpha receptor) and Cy5-labeled strepavidin.
Figure 3 shows the effect in the competitive inhibition of TNF-α/TNF-αR binding of two selected samples (fractions 4 and 5 of Herb C corresponding to the spots located at B7 and B8, and B9 and B 10 of the herbal chip shown in Figure 2A) that showed green fluorescence (Cy3) signal.

### Detailed Description of the Invention

The present invention discloses a new platform, named herbal chip, for shotgun screening for active ingredients contained in herbs in a target-directed manner for achieving high throughput. The herbal chip of the present invention comprises an herbal chip comprising a plastic slide, a coating on the plastic slide which binds fractions or components obtained from herbs to said slide in independently allocated microarrays on the coating wherein the plastic slide is pre-treated with a polyfunctional aldehyde followed by soaking in a solution of NH₂ group(s) - providing precursor before coating the plastic slide, and wherein said coating is made of a polyfunctional epoxide compound linking the components contained in herbs to the pre-treated plastic slide.

In the herbal chip of the present invention, each of the fractions or components obtained from herbs that is spotted in microarrays is substantially an uncharacterized, homogeneous or heterogeneous, partially-purified mixture obtained from the herbs. The fractions or components may be obtained by fractionating an extract of the herb by applying an apparatus, e.g. HPLC. Each of the spots on the herbal chip may comprise secondary metabolites of the herb.

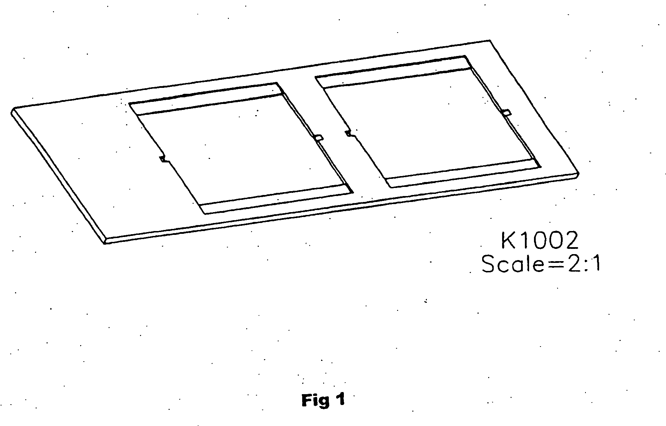

In the herbal chip of the present invention, a plastic slide is used in place of the conventional glass slide. The material of the plastic slide is a homopolymer or copolymer, which is made of one or more monomers selected from the group consisting of ethylene, haloethylene, propylene, halopropylene, acrylate, methacrylate, butadiene, acrylonitrile, norbomene and styrene, wherein a polymer of styrene is preferred. Also included in the material of the plastic slide is polycarbonate. The plastic slide is comparable in size to the ones conventionally used within a microarrayer and a laser scanner. The advantage of using a plastic slide in the herbal chip of the present invention is that there are a variety of chemicals that can be used for treating the surface of a plastic slide, whereby not only macromolecules (such as proteins and DNA) but also micromolecules (such as metabolites of herbs) can be immobilized on the surface of the plastic slide, in view of the fact that conventional glass slide was used for immobilizing just macromolecules, such as proteins and DNA. Further, a plastic slide can be easily molded into a shape as desired and is also effective in cost. In an embodiment of the present invention, the plastic slide is molded with two cavity chambers as shown in Figure 1, based on which samples obtained from fractions or components of a herb can be gridded on the surface of the chambers, and a probe(s)-containing solution for conducting hybridization is then loaded onto the chambers. The depth of the two cavity chambers may be the same or different, and ranges from less than 0.03 mm to up to 0.5 mm. Further, as shown in Figure 1, two bars are respectively molded at the opposite sides of each chamber for supporting a glass lid, wherein the glass lid is useful for preventing the evaporation or loss of the probe(s)-containing solution loaded onto the chamber.

In the herbal chip of the present invention, the plastic slide is pretreated with a polyfunctional aldehyde followed by soaking in a solution of NH₂ group(s)-providing precursor, whereby the resultant plastic slide contains active amino groups on its surface. The NH₂ group(s)-providing precursor may be organic or inorganic, and may be selected from the group consisting of NH₄OH, primary amine, secondary amine and tertiary amine, wherein the aliphatic or aromatic part of the primary amine, secondary amine and tertiary amine may be useful as an additional spacer arm. Among the NH₂ group(s)-providing precursors, NH₄OH directly providing free NH₂ group is preferred.

In the herbal chip of the present invention, the coating on the plastic slide is made of polyfunctional epoxides, as a spacer. The polyfunctional epoxides act for linking the components contained in herbs to the pretreated plastic slide. The active epoxy groups on one end of the polyfunctional epoxides react with the amino groups on the surface of the pretreated plastic slide, while active epoxy groups on the other end of the polyfunctional epoxides react with or absorb ingredients contained in the herbs. In particular, those molecules in the ingredients of herbs that contain free hydroxyl, sulfhydryl or amino groups can form a covalent bond with the active epoxy groups on the other end of the polyfunctional epoxides, and consequently are attached onto the plastic slide. The polyfunctional epoxides preferably contain a long chemical chain of 6 to 24 carbon atoms, whereby the ingredients of herbs would not directly bind to the pretreated plastic slide. In the herbal chip of the present invention, the binding of each spot on the coated plastic slide is persistent, even after stringent stripping. In the present invention, not only macromolecules (such as proteins and DNA) but also small molecules (such as metabolites of herbs) can be immobilized in a homogeneous or heterogeneous manner on the surface of the coated plastic slide.

The preparation of the herbal chip of the present invention comprises the steps of preparing a plastic slide preferably provided with cavity chambers, pretreating the plastic slide with a polyfunctional aldehyde followed by soaking in a solution of NH₂ group(s)-providing precursor (preferably, aqueous ammonia), coating the surface of the pretreated plastic slide with polyfunctional epoxides and spotting and immobilizing on the coated plastic slide a massive amount of samples in a gridded area in microarrays with a microarrayer by applying the high-density gridding technology, wherein each of sample spots contains homogeneous or heterogeneous fractions or ingredients obtained from a herb. In the preparation of the herbal chip of the present invention, the integrating miniaturization technique can be used for increasing the density of samples gridded on the coated plastic slide.

The present invention also discloses a method of using the herbal chip for screening for active ingredients contained in herbs based on target-directed strategy, comprising the steps of loading a labeled probe(s)-containing solution onto the herbal chip (e.g. the chambers of the herbal chip) for conducting hybridization (wherein each of the chambers may be covered by a glass lid for preventing the evaporation of the labeled probe(s)-containing solution), and imaging and identifying the spots that react with or bind to the labeled probe with an apparatus, e.g. a laser scanner. The label within the probes may a dye or a radioactive material.

The probes used in the present invention are homogeneous or heterogeneous, known targets based on a defined molecular mechanism, which may be, for example, small molecules, competitive ligands, or antibodies against, for example, the selected cells, receptors, enzymes, or proteins. In an embodiment of the present invention, tumor necrosis factor-alpha receptor (TNF-αR) labeled with Cy3 and strepavidin labeled with Cy5 were used as probes for conducting hybridization. Thus, for example, if a signal indicating the binding of ingredients in a spot on the herbal chip with the labeled 7NF-αR is observed, there should be at least one candidates in the ingredients of the spot that exhibit a biological activity similar to anti-TNF-αR antibody. Those candidates may be useful as an antagonist for diminishing inflammatory response and then for treating autoimmune diseases, such as rheumatoid arthritis.

The herbal chip of the present invention significantly increases the throughput in the shotgun screening of biologically active ingredients contained in herbs based on the advantage that thousands of samples can be simultaneously tested. By applying the herbal chip of the present invention, any ingredient contained in herbs that exhibits a potentially pharmacological or therapeutic effect can be quickly detected, isolated and identified.

The following Example is provided to further illustrate the present invention, but the scope of the present invention should not be limited to the following Example.

### Example

### Pretreatment of the plastic slide and preparation of the coated plastic slide

The molded plastic slide shown in Figure 1 was made of a polymer of styrene and comprised two cavity chambers. The molded plastic slide was comparable in size with regular glass slides used in a microscope or laser scanner, wherein the depth of each of the cavity chambers is 0.05 mm.

The molded plastic slide was first immersed in 0.4 % glutaldehyde solution (pH 5.0) for 4 hours at room temperature, followed by washing with water and then soaking in 3M NH₄OH (pH 11.0) at 60°C for 4 hours. The resultant plastic slide was treated with 100 mM 1,4-butanediol diglycidyl ether (pH 11.0) at 37°C overnight. Finally, the plastic slide was washed with 0.1 M NaHCO₃ (pH 8.0) and then was dried.

### Loading samples onto the coated plastic slide in microarray format

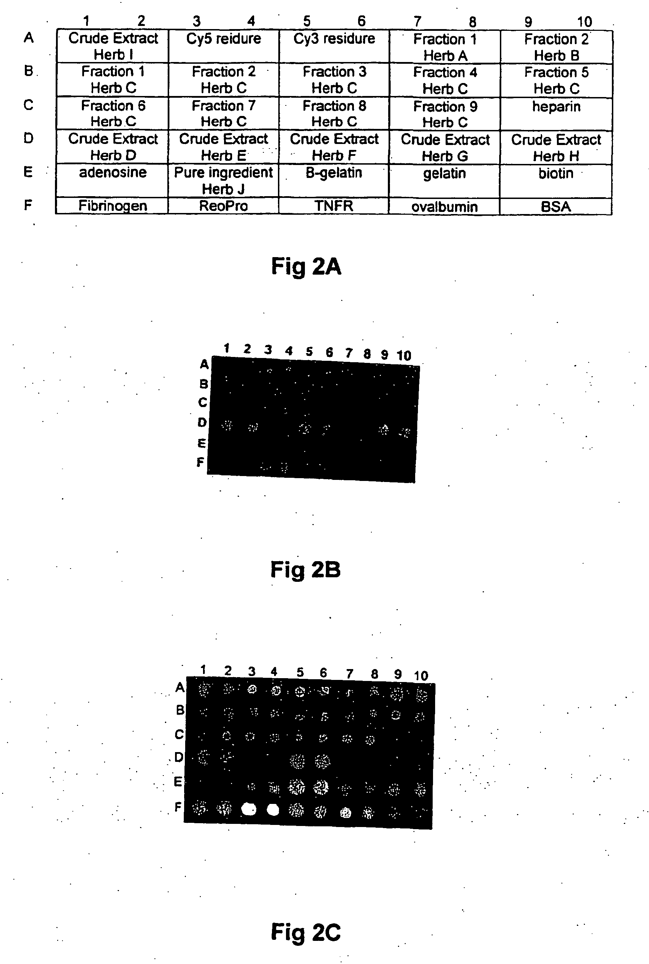

Microarrayer BioGrid (purchased from BioRobotic, Cambridge, UK) was applied to spot samples onto the above coated plastic slide. Samples were first dissolved and dispensed in 96-well microplates. A 4-pin (0.2 µm) tool was used to load samples from the 96-well microplates onto the surface of the cavity chambers of the coated plastic slide continuously, wherein the samples obtained from herbs as shown in Figure 2A are illustrated as follows: Herb A and B were different fractions obtained from *Taraxacum mongolicum,* Herb G was a crude extract of *Taraxacum mongolicum,* Herb F was a crude extract of *Hedyotis diffuse*, Fractions 1 to 9 of Herb C were different, partially purified fractions obtained from *Hedyotis diffuse*, Herb D was a crude extract of *Lonicera japonica*, Herb E was a crude extract of *Carthamus tinctorius,* Herb J was a pure ingredient obtained from *Carthamus tinctorius,* Herb H was a crude extract of *Forsythia suspensa*, and Herb I was a crude extract of *Paeonia lactiflora*. After the spots on the surface of the cavity chambers of the plastic slide were dried, the plastic slide was soak-treated with 1M Tris (pH 8.0) at 37°C for 2 hours. The resultant plastic slide was then imaged by a laser scanner (Axon, USA) to make sure that all of the samples were adsorbed onto the plastic slide (see Figure 2B).

### Hybridization and signal detection

Tumor necrosis factor-alpha receptor (TNF-αR) labeled with Cy3 and strepavidin labeled with Cy5 were used as probes for conducting hybridization. Two glass lids were used to respectively cover the two cavity chambers of the plastic slide prior to loading 100 µl of hybridization solution (TBST buffer containing 50 mM Tris, pH 7.3, 0.15 M NaCl, and 0.02 % Tween 20) containing both of the labeled probes. The plastic slide was then allowed to stand at room temperature for 4 hours, followed by washing with TBST buffer 3 times and then with water 3 times. Finally, the plastic slide was dried at 37°C . The plastic slide was imaged by the laser scanner (Axon, USA). The image result was showed in Figure 2C.

The green fluorescent spots on the image of Figure 2C that showed inhibition in the TNF-α/TNF-αR binding were collected. Those fractions obtained from herbs corresponding to the green fluorescent spots contained active ingredients that could bind with TNF-αR.

### Biological assay of the samples showing positive green fluorescent signal

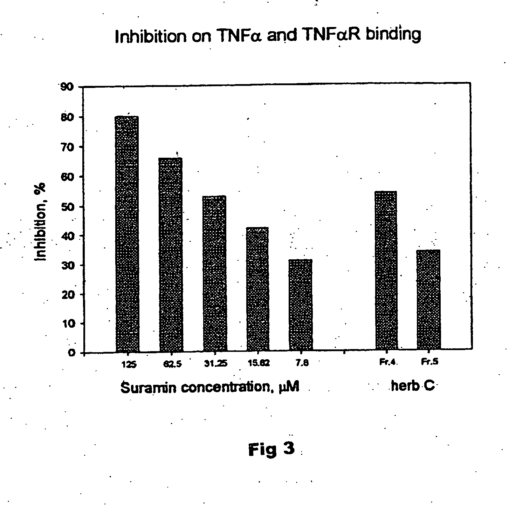

For demonstrating the biological activity of the fractions as collected above in the inhibition of TNF-α/TNF-αR binding, an assay as described in the report of Mancini et al., Biochemical Pharmacology 58: 851-859, 1999 was performed, wherein a known substance, Suramin, was used as a positive control and two samples (fractions 4 and 5 of Herb C corresponding to the spots located at B7 and B8, and B9 and B10 of the herbal chip shown in Figure 2A) showing green fluorescence were tested. The result was shown in Figure 3.

## Claims

1. An herbal chip comprising a plastic slide, a coating on the plastic slide which binds fractions or components obtained from herbs to said slide in independently allocated microarrays on the coating wherein the plastic slide is pre-treated with a polyfunctional aldehyde followed by soaking in a solution of NH₂ group(s)-providing precursor before coating the plastic slide, and wherein said coating is made of a polyfunctional epoxide compound linking the components contained in herbs to the pre-treated plastic slide.

2. The herbal chip as claimed in claim 1, wherein the fractions or components obtained from herbs are homogenous or heterogenous.

3. The herbal chip as claimed in claim 1, wherein the fractions or components obtained from herbs are obtained by fractionating an extract of the herb by applying HPLC.

4. The herbal chip as claimed in claim 1, wherein the fractions or components obtained from herbs contain secondary metabolites of a herb.

5. The herbal chip as claimed in claim 1, wherein the material of the plastic slide is a polycarbonate, or a homopolymer or copolymer that is made of one or more monomers selected from the group consisting of ethylene, haloethylene, propylene, halopropylene, acrylate, methacrylate, butadiene, acrylonitrile, norbomene and styrene.

6. The herbal chip as claimed in claim 5, wherein the plastic slide is made of a polymer of styrene.

7. The herbal chip as claimed in claim 1, wherein the plastic slide has two cavity chambers.

8. The herbal chip as claimed in claim 1, wherein the polyfunctional aldehyde is glutaldehyde.

9. The herbal chip as claimed in claim 1, wherein the NH₂ group(s)-providing compound is NH₄OH.

10. The herbal chip as claimed in claim 1, wherein the polyfunctional epoxide contains at least one epoxy group at each of its ends.

11. The herbal chip as claimed in claim 10, wherein the epoxy group(s) at one end of the polyfunctional epoxide react with the NH₂ group(s) on the surface of the pre-treated plastic slide.

12. The herbal chip as claimed in claim 10, wherein the epoxy group(s) at the other end of the polyfunctional epoxide react with the free hydroxyl, sulfhydryl or amino groups of the ingredients contained in herbs.

13. The herbal chip as claimed in claim 10, wherein the polyfunctional epoxide contains a long chemical chain of 6 to 24 carbon atoms.

14. A method of producing the herbal chip as claimed in claim 1, comprising the step of coupling the herbal fractions or components to said epoxide contained in said coating for coupling to an herbal fraction or component.

15. The method as claimed in claim 14, wherein the plastic slide has two cavity chambers and the samples are spotted or immobilized on the surface of the cavity chambers.

16. The method as claimed in claim 14, wherein said coupling is preceded by the steps of treating the slide with said polyfunctional aldehyde followed by soaking in a solution of said NH₂-providing compound.

17. The method as claimed in claim 16, wherein the polyfunctional aldehyde is glutaldehyde.

18. The method as claimed in claim 16, wherein the NH₂-providing precursor is NH₄OH.

19. The method as claimed in claim 14, wherein the polyfunctional epoxide compound contains at least one epoxy group at each of its ends.

20. A method of using the herbal chip as claimed in claim 1 for screening for active ingredients contained in herbs, comprising the steps of loading a labeled probe(s)-containing solution onto the herbal chip for conducting hybridization, and imaging and identifying the gridded samples that react with or bind to the labeled probe.

21. The method as claimed in claim 20, wherein the labeled probe(s)-containing solution is homogenous or heterogenous.

22. The method as claimed in claim 20, wherein the label is a dye or a radioactive material.

## Patentansprüche

1. Herbaler Chip, umfassend einen Kunststoffobjektträger, eine Beschichtung auf dem Kunststoffobjektträger, die aus krautigen Pflanzen erhaltene Fraktionen oder Komponenten an den Objektträger in unabhängig verteilten Mikroarrays auf der Beschichtung bindet, wobei der Plastikobjektträger mit einem polyfunktionellen Aldehyd vorbehandelt ist, gefolgt von einem Eintauchen in eine Lösung eines NH₂-Gruppe(n)-bereitstellenden Vorläufers, vor dem Beschichten des Kunststoffobjektträgers, und wobei die Beschichtung aus einer polyfunktionellen Epoxidverbindung hergestellt ist, die die in den krautigen Pflanzen enthaltenen Komponenten mit dem vorbehandelten Kunststoffobjektträger verknüpft.

2. Herbaler Chip nach Anspruch 1, wobei die aus den krautigen Pflanzen erhaltenen Fraktionen oder Komponenten homogen oder heterogen sind.

3. Herbaler Chip nach Anspruch 1, wobei die aus den krautigen Pflanzen erhaltenen Fraktionen oder Komponenten durch Fraktionieren eines Extrakts aus der krautigen Pflanze durch Anwenden von HPLC erhalten wurden.

4. Herbaler Chip nach Anspruch 1, wobei die aus den krautigen Pflanzen erhaltenen Fraktionen oder Komponenten Sekundärmetabolite einer krautigen Pflanze enthalten.

5. Herbaler Chip nach Anspruch 1, wobei das Material des Kunststoffobjektträgers ein Polycarbonat oder ein Homopolymer oder ein Copolymer ist, das aus einem oder mehreren Monomeren, ausgewählt aus der Gruppe bestehend aus Ethylen, Halogenethylen, Propylen, Halogenpropylen, Acrylat, Methacrylat, Butadien, Acrylonitril, Norbornen und Styrol, hergestellt wurde.

6. Herbaler Chip nach Anspruch 2, wobei der Kunststoffobjektträger aus einem Styrolpolymer hergestellt ist.

7. Herbaler Chip nach Anspruch 1, wobei der Kunststoffobjektträger zwei Vertiefungskammern hat.

8. Herbaler Chip nach Anspruch 1, wobei der polyfunktionelle Aldehyd Glutaraldehyd ist.

9. Herbaler Chip nach Anspruch 1, wobei die NH₂-Gruppe(n)-bereitstellende Verbindung NH₄OH ist.

10. Herbaler Chip nach Anspruch 1, wobei das polyfunktionelle Epoxid mindestens eine Epoxygruppe an jedem seiner Enden enthält.

11. Herbaler Chip nach Anspruch 10, wobei die Epoxygruppe(n) an einem Ende des polyfunktionellen Epoxids mit der/den NH₂₋Gruppe(n) auf der Oberfläche des vorbehandelten Kunststoffobjektträgers reagiert/reagieren.

12. Herbaler Chip nach Anspruch 10, wobei die Epoxygruppe(n) an dem anderen Ende des polyfunktionellen Epoxids mit den freien Hydroxyl-, Sulfhydryl- oder Aminogruppen der in krautigen Pflanzen enthaltenen Inhaltsstoffe reagiert/reagieren.

13. Herbaler Chip nach Anspruch 10, wobei das polyfunktionelle Epoxid eine lange chemische Kette von 6 bis 24 Kohlenstoffatomen enthält.

14. Verfahren zum Herstellen des herbalen Chips nach Anpruch 1, umfassend den Schritt des Koppelns der herbalen Fraktionen oder Komponenten an das Epoxid, das in der Beschichtung enthalten ist, um an eine herbale Fraktion oder Komponente zu koppeln.

15. Verfahren nach Anspruch 14, wobei der Kunststoffobjektträger zwei Vertiefungskammern hat und die Proben auf die Oberfläche der Vertiefungskammern aufgetüpfelt oder darauf immobilisiert werden.

16. Verfahren nach Anspruch 14, wobei dem Koppeln die Schritte des Behandelns des Objektträgers mit dem polyfunktionellen Aldehyd, gefolgt von Eintauchen in eine Lösung der NH₂-bereitstellenden Verbindung, vorausgehen.

17. Verfahren nach Anspruch 16, wobei der polyfunktionelle Aldehyd Glutaraldehyd ist.

18. Verfahren nach Anspruch 16, wobei der NH₂-bereitstellende Vorläufer NH₄OH ist.

19. Verfahren nach Anspruch 14, wobei die polyfunktionelle Epoxidverbindung mindestens eine Epoxygruppe an jeder ihrer Enden enthält.

20. Verfahren zum Verwenden des herbalen Chips nach Anspruch 1, zum Screening nach in krautigen Pflanzen enthaltenen aktiven Inhaltsstoffen, umfassend die Schritte des Aufgebens einer markierten Prüfsubstanz(en)-enthaltenden Lösung auf den herbalen Chip zum Durchführen der Hybridisierung und das Sichtbarmachen und Identifizieren der gitterförmig angeordneten Proben, die mit der markierten Prüfsubstanz reagieren oder an sie binden.

21. Verfahren nach Anspruch 20, wobei die markierte Prüfsubstanz(en)-enthaltende Lösung homogen oder heterogen ist.

22. Verfahren nach Anspruch 20, wobei die Markierung ein Farbstoff oder ein radioaktives Material ist.

## Revendications

1. Puce à base d'herbes comprenant une lamelle plastique, un revêtement sur la lamelle plastique qui fixe des fractions ou des composants provenant d'herbes à ladite lamelle en micro-agencements disposés de manière indépendante sur le revêtement, dans laquelle la lamelle plastique est pré-traitée par un aldéhyde polyfonctionnel, opération suivie d'un trempage dans une solution de précurseurs fournissant un ou plusieurs groupes NH₂ avant le revêtement de la lamelle plastique, et dans laquelle ledit revêtement est formé d'un composé époxyde polyfonctionnel liant les composants contenus dans les herbes à la lamelle plastique pré-traitée.

2. Puce à base d'herbes selon la revendication 1, dans laquelle les fractions ou les composants provenant d'herbes sont homogènes ou hétérogènes.

3. Puce à base d'herbes selon la revendication 1, dans laquelle les fractions ou composants provenant d'herbes sont obtenus par fractionnement d'un extrait de l'herbe par application d'une CLHP.

4. Puce à base d'herbes selon la revendication 1, dans laquelle les fractions ou composants provenant d'herbes contiennent des métabolites secondaires d'une herbe .

5. Puce à base d'herbes selon la revendication 1, dans laquelle le matériau de la lamelle plastique est un polycarbonate, ou un homopolymère ou copolymère qui est formé d'un ou plusieurs monomères choisis dans le groupe constitué par l'éthylène, un halogénoéthylène, le propylène, un halogénopropylène, un acrylate, un méthacrylate, le butadiène, l'acrylonitrile, le norbornène et le styrène.

6. Puce à base d'herbes selon la revendication 5, dans laquelle la lamelle plastique est formée d'un polymère de styrène.

7. Puce à base d'herbes selon la revendication 1, dans laquelle la lamelle plastique comporte deux chambres formant des cavités.

8. Puce à base d'herbes selon la revendication 1, dans laquelle l'aldéhyde polyfonctionnel est le glutaldéhyde.

9. Puce à base d'herbes selon la revendication 1, dans laquelle le composé fournissant un ou plusieurs groupes NH₂ est NH₄OH.

10. Puce à base d'herbes selon la revendication 1, dans laquelle l'époxyde polyfonctionnel contient au moins un groupe époxy à chacune de ses extrémités.

11. Puce à base d'herbes selon la revendication 10, dans laquelle le ou les groupes époxy à une extrémité de l'époxyde polyfonctionnel réagissent avec le ou les groupes NH₂ sur la surface de la lamelle plastique pré-traitée.

12. Puce à base d'herbes selon la revendication 10, dans laquelle le ou les groupes époxy à l'autre extrémité de l'époxyde fonctionnel réagissent avec les groupes hydroxyle, sulfhydryle ou amino libres des ingrédients contenus dans les herbes .

13. Puce à base d'herbes selon la revendication 10, dans laquelle l'époxyde polyfonctionnel contient une longue chaîne chimique comportant de 6 à 24 atomes de carbone.

14. Procédé pour produire la puce à base d'herbes selon la revendication 1, comprenant l'étape de couplage des fractions ou composants d'herbes audit époxyde contenu dans ledit revêtement pour le couplage à une fraction ou un composant d'herbes .

15. Procédé selon la revendication 14, dans laquelle la lamelle plastique comporte deux chambres formant des cavités et les échantillons sont mouchetés ou immobilisés sur la surface des chambres formant des cavités.

16. Procédé selon la revendication 14, dans lequel ledit couplage est précédé des étapes de traitement de la lamelle avec ledit aldéhyde polyfonctionnel suivi d'un trempage dans une solution dudit composé fournissant NH₂.

17. Procédé selon la revendication 16, dans lequel l'aldéhyde polyfonctionnel est le glutaldéhyde.

18. Procédé selon la revendication 16, dans lequel le précurseur fournissant NH₂ est NH₄OH.

19. Procédé selon la revendication 14, dans lequel le composé époxyde polyfonctionnel contient au moins un groupe époxy à chacune de ses extrémités.

20. Procédé pour utiliser la puce à base d'herbes de la revendication 1 en vue de dépister des ingrédients actifs contenus dans des herbes, comprenant les étapes de chargement d'une solution, contenant une ou plusieurs sondes marquées, sur la puce à base d'herbes en vue de réaliser une hybridation, puis de représentation par une image et d'identification des échantillons grillagés qui réagissent ou se fixent avec la sonde marquée.

21. Procédé selon la revendication 20, dans lequel la solution contenant une ou plusieurs sondes marquées est homogène ou hétérogène.

22. Procédé selon la revendication 20, dans lequel le marqueur est un colorant ou un matériau radioactif.
